# EUROPEAN PATENT APPLICATION

(11) **EP 4 767 925 A1**
(43) Date of publication of application: **01.07.2026**
(21) Application number: 25205107.3
(22) Date of filing: 26.09.2025
(51) Int. Cl.: A61B 5/00, A61B 5/06, G06T 17/00

(54) **BRAIN FUNCTIONAL AREA LOCATION METHOD, WEARABLE EDGE COMPUTING DEVICE, AND STORAGE MEDIUM**

(30) Priority: 27.12.2024 CN 202411960408; 31.12.2024 CN 202411998581
(71) Applicant: Kingfar International Inc., Beijing 100080 (CN); Nanjing Kingfar Health Technology Inc., Nanjing, Jiangsu 211112 (CN)
(72) Inventor: ZHAO, Qichao, BEIJING, 100080 (CN); WANG, Qingju, BEIJING, 100080 (CN); YANG, Ran, BEIJING, 100080 (CN)
(74) Representative: DREISS Patentanwälte PartG mbB

(57) **Abstract**

Provided are a brain functional area location method, a wearable edge computing device, and a storage medium. The includes: obtaining first video data and second video data which are a multi-angle head video recorded when a user's head is not wearing a wearable apparatus and a multi-angle head video recorded when the user's head is wearing the wearable apparatus, respectively; obtaining a first three-dimensional model and a second three-dimensional model of the user's head based on the first video data and the second video data, respectively; obtaining relative position information between a detection device in the wearable apparatus and the user's head based on the first three-dimensional model and the second three-dimensional model; and registering the first three-dimensional model with a predetermined standard spatial template, and obtaining a brain area position of the user corresponding to the detection device based on a registration result and the relative position information.

## Description

### FIELD

The present disclosure relates to the field of data processing technologies, and more particularly, to a brain functional area location method, a wearable edge computing device, and a computer-readable storage medium.

### BACKGROUND

Currently, the main methods for obtaining brain cognitive signals with high ecological validity are electroencephalogram (EEG) and functional near-infrared spectroscopy (fNIRS) imaging. However, due to individual differences, each person has a unique head shape, leading to variations in the specific brain area positions measured, even when individuals wear devices with the same channels and electrodes in the same manner. In addition, the above-described methods require combining magnetic field technology to collect a position of each channel. However, obtaining of Nuclear Magnetic Resonance (MRI) signals is relatively costly and inconvenient.

For tNIRS, after the localization of brain functional areas are completed, it is necessary to conduct quality evaluation of fNIRS functional optical brain imaging signals to ensure the reliability of brain functional imaging. However, current evaluations of such signals mostly focus on evaluating the stability of optical signals, which fails to comprehensively measure the quality of fNIRS functional optical brain imaging signals and is unable to adapt to the physiological characteristics of different individuals.

### SUMMARY

The present disclosure aims to solve at least one of the technical problems in the related art to a certain extent.

To this end, a first objective of the present disclosure is to provide a brain functional area location method to improve accuracy of brain area localization, with low requirements for devices, low costs, and ease for wide application.

A second objective of the present disclosure is to provide a wearable edge computing device.

A third objective of the present disclosure is to provide a computer-readable storage medium.

In a first aspect, embodiments of the present disclosure provide a brain functional area location method. The method includes: obtaining first video data and second video data, the first video data being a multi-angle head video recorded when a user's head is not wearing a wearable apparatus, and the second video data being a multi-angle head video recorded when the user's head is wearing the wearable apparatus; obtaining a first three-dimensional model of the user's head based on the first video data, and obtaining a second three-dimensional model of the user's head based on the second video data; obtaining relative position information between a detection device in the wearable apparatus and the user's head based on the first three-dimensional model and the second three-dimensional model; and registering the first three-dimensional model with a predetermined standard spatial template, and obtaining a brain area position of the user corresponding to the detection device based on a registration result and the relative position information.

With the brain functional area location method according to the embodiments of the present disclosure, multi-angle head videos recorded when the user's head is not wearing the wearable apparatus and when the user's head is wearing the wearable apparatus are obtained. The first three-dimensional model of the user's head is obtained based on the first video data, and the second three-dimensional model of the user's head is obtained based on the second video data. The relative position information between the detection device in the wearable apparatus and the user's head is obtained based on the first three-dimensional model and the second three-dimensional model. The first three-dimensional model is registered with the predetermined standard spatial template, and the brain area position of the user corresponding to the detection device is obtained based on the registration result and the relative position information. During this process, real shape of the user's head is obtained, and registration with predetermined standard spatial template is employed, which can improve accuracy of brain area localization. Also, this process does not require complex devices and has low device requirements, resulting in low implementation costs and facilitating its popularization and application.

According to an embodiment of the present disclosure, said obtaining the first three-dimensional model of the user's head based on the first video data includes: extracting head feature points of the user from each frame in the first video data; performing feature matching on the head feature points to obtain a shape and motion information of the user's head; and obtaining the first three-dimensional model based on the shape and the motion information.

According to an embodiment of the present disclosure, when performing the feature matching on the head feature points, a random sampling consensus algorithm is further used to remove incorrect matching points.

According to an embodiment of the present disclosure, the first three-dimensional model includes predetermined head marker points of the user, and the second three-dimensional model includes the predetermined head marker points and a detection device marker point. Said obtaining the relative position information between the detection device in the wearable apparatus and the user's head based on the first three-dimensional model and the second three-dimensional model includes: registering the first three-dimensional model and the second three-dimensional model based on the predetermined head marker points to obtain a position of the detection device on a scalp surface of the user; and obtaining the relative position information based on the position of the detection device on the scalp surface of the user and the predetermined head marker points.

According to an embodiment of the present disclosure, said obtaining the relative position information based on the position of the detection device on the scalp surface of the user and the predetermined head marker points includes: determining three-dimensional coordinates of the position of the detection device on the scalp surface of the user and the predetermined head marker points; and obtaining the relative position information based on the three-dimensional coordinates and positions of the predetermined head marker points on the user's head.

According to an embodiment of the present disclosure, the predetermined head marker points include at least two of a nasion position, bilateral ear canal mastoid positions, and a posterior cranial protuberance position of the user.

According to an embodiment of the present disclosure, said registering the first three-dimensional model with the predetermined standard spatial template, and obtaining the brain area position of the user corresponding to the detection device based on the registration result and the relative position information includes: voxelizing the first three-dimensional model, and converting the voxelized first three-dimensional model into the same voxel scale as the predetermined standard spatial template; mapping voxels of the first three-dimensional model to the predetermined standard spatial template using a non-rigid registration method; and obtaining the brain area position of the user corresponding to the detection device based on a mapping result and the relative position information.

According to an embodiment of the present disclosure, the wearable apparatus includes a wearable fNIRS functional optical brain imaging device. The detection device includes an fNIRS light source. The method further includes: obtaining incident light intensity data and emitting light intensity data that are emitted by the fNIRS light source corresponding to the brain area position of the user; determining a coefficient of variation based on the incident light intensity data, and determining a signal-to-noise ratio based on the incident light intensity data and the emitting light intensity data; processing the incident light intensity data to obtain the number of peaks within a predetermined frequency range and a heartbeat signal feature; and determining a quality evaluation result of an fNIRS functional optical brain imaging signal based on the coefficient of variation, the signal-to-noise ratio, the number of peaks, and the heartbeat signal feature.

According to an embodiment of the present disclosure, the heartbeat signal feature includes an average value of inter-beat intervals, an average heart rate, a standard deviation of normal-to-normal intervals, as well as a cross-correlation coefficient and peak power between red light and infrared light corresponding to a heartbeat signal. Obtaining the heartbeat signal feature includes: converting the incident light intensity data into optical density data, filtering chromatic light at an infrared wavelength and performing R-point detection, and calculating the average value of inter-beat intervals, the average heart rate, and the standard deviation of normal-to-normal intervals by means of a sliding window approach.

According to an embodiment of the present disclosure, said obtaining the number of peaks within the predetermined frequency range includes: performing Fourier transform on the incident light intensity data to obtain a frequency domain signal of the incident light intensity data; ranking amplitudes of the frequency domain signal within the predetermined frequency range in descending order; and taking a predetermined number of amplitudes that are ranked top as peaks, and obtaining the number of peaks.

According to an embodiment of the present disclosure, said determining the quality evaluation result of the fNIRS functional optical brain imaging signal based on the coefficient of variation, the signal-to-noise ratio, the number of peaks, and the heartbeat signal feature includes: determining a first signal quality index based on the coefficient of variation, the coefficient of variation being negatively correlated with the first signal quality index; determining a second signal quality index based on the number of peaks and a predetermined number of peaks, a difference between the number of peaks and the predetermined number of peaks being negatively correlated with the second signal quality index; determining a third signal quality index based on the average value of inter-beat intervals, the average heart rate, and a first standard deviation of normal-to-normal intervals, an average value of reference inter-beat intervals, a reference average heart rate, and a second standard deviation of reference normal-to-normal intervals, and determining a fourth signal quality index based on a cross-correlation coefficient difference between the cross-correlation coefficient and a predetermined cross-correlation coefficient, a peak power difference between the peak power and a predetermined peak power threshold, where a target difference is determined using correlation and variance analysis based on the first standard deviation and the second standard deviation, the target difference is negatively correlated with the third signal quality index, and the cross-correlation coefficient difference and the peak power difference are each positively correlated with the fourth signal quality index; determining a fifth signal quality index based on the signal-to-noise ratio, the signal-to-noise ratio being positively correlated with the fifth signal quality index; determining a first product of the first signal quality index and a corresponding weight coefficient, a second product of the second signal quality index and a corresponding weight coefficient, a third product of the third signal quality index and a corresponding weight coefficient, a fourth product of the fourth signal quality index and a corresponding weight coefficient, and a fifth product of the fifth signal quality index and a corresponding weight coefficient; and determining the quality evaluation result of the fNIRS functional optical brain imaging signal based on a sum of the first product, the second product, the third product, the fourth product and the fifth product, the evaluation result being positively correlated with each of the first signal quality index, the second signal quality index, the third signal quality index, the fourth signal quality index and the fifth signal quality index.

According to an embodiment of the present disclosure, the method further includes: adjusting the signal-to-noise ratio when the quality evaluation result of the fNIRS functional optical brain imaging signal is smaller than a predetermined result threshold.

According to an embodiment of the present disclosure, said adjusting the signal-to-noise ratio includes: increasing intensity of infrared light emitted by the fNIRS light source.

According to an embodiment of the present disclosure, the method further includes: obtaining head data of a test subject; and obtaining hair density based on the head data, and increasing the intensity of the infrared light emitted by the fNIRS light source when the hair density is high.

In a second aspect, embodiments of the present disclosure provide a wearable edge computing device. The device includes: a memory; and a processor. The memory has a computer program stored thereon. The processor implements, when executing the computer program, the method described in the first aspect.

In a third aspect, embodiments of the present disclosure provide a computer-readable storage medium, having a computer program stored thereon. The computer program, when executed by a processor, implements the method described in the first aspect.

### BRIEF DESCRIPTION OF THE DRAWINGS

FIG. 1 is a flowchart of a brain functional area location method according to an embodiment of the present disclosure.
FIG. 2 is a schematic diagram of second video data according to an example of the present disclosure.
FIG. 3 is a flowchart of another brain functional area location method according to an embodiment of the present disclosure.
FIG. 4 is a flowchart of a method for evaluating quality of an fNIRS functional optical brain imaging signal in a brain functional area location method according to an embodiment of the present disclosure.
FIG. 5 is a flowchart of another method for evaluating quality of an fNIRS functional optical brain imaging signal in a brain functional area location method according to an embodiment of the present disclosure.
FIG. 6 is a structural block diagram of a wearable edge computing device according to an embodiment of the present disclosure.

### DETAILED DESCRIPTION

Embodiments of the present disclosure will be described in detail below with reference to examples thereof as illustrated in the accompanying drawings, throughout which same or similar elements, or elements having same or similar functions, are denoted by same or similar reference numerals. The embodiments described below with reference to the drawings are illustrative only, and are intended to explain, rather than limit, the present disclosure.

At present, a common method to obtain a specific scalp position corresponding to a channel (which can correspond to an electroencephalographic electrode) on an electroencephalographic signal collection device involves using a magnetic field locating pen to record the position by contacting a position of the channel on the scalp before and after measurement. However, this method has limited applicability to devices and is not suitable for device where a detector (such as the electroencephalographic electrode) does not directly contact the scalp, such as a high-density channel collection device. In addition, if individual differences are to be eliminated for achieving accurate brain area location, it is necessary to scan an individual's brain structure through nuclear magnetic resonance technology, which is costly. To this end, the present disclosure provides a universal brain area location method, which is realized by image recognition, image registration, model voxelization, etc.

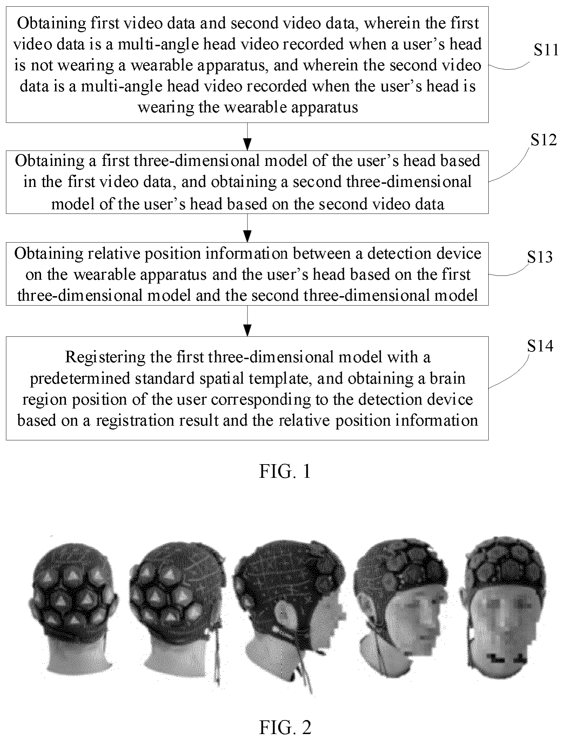

FIG. 1 is a flowchart of a brain functional area location method according to an embodiment of the present disclosure.

As illustrated in FIG. 1, the brain functional area location method includes the operations.

At S11, first video data and second video data are obtained. The first video data is a multi-angle head video recorded when a user's head is not wearing a wearable apparatus, and the second video data is a multi-angle head video recorded when the user's head is wearing the wearable apparatus.

**In** an exemplary embodiment of the present disclosure, locating markers can be placed at at least two of a nasion position, bilateral ear canal mastoid positions, and a posterior cranial protuberance position of the user, respectively, to serve as predetermined head marker points. Also, a locating marker can be placed on the wearable apparatus at a channel (or the position of a light source and a detector) as a detection device marker point. When the user's head is not wearing the wearable apparatus (such as the above-described electroencephalographic signal collection device), the multi-angle head video of the user is recorded using a camera, a smartphone, or the like. As illustrated in FIG. 2, when the user's head is wearing the wearable apparatus, the multi-angle head video of the user is recorded using the camera, the smartphone, or the like (FIG. 2 shows video frames recorded from five angles when the user's head is wearing the wearable apparatus. The five angles are a posterior side, a right posterior side, a right side, a right anterior side, and an anterior side, respectively. Three corners of triangles in the figure correspond to the above-described positions of the light source or detector).

At S12, a first three-dimensional model of the user's head is obtained based on the first video data, and a second three-dimensional model of the user's head is obtained based on the second video data.

**In** some embodiments of the present disclosure, said obtaining the first three-dimensional model of the user's head based on the first video data includes: extracting head feature points of the user from each frame in the first video data; performing feature matching on the head feature points to obtain a shape and motion information of the user's head; and obtaining the first three-dimensional model based on the shape and the motion information.

In an exemplary embodiment of the present disclosure, feature extraction algorithms such as Scale-Invariant Feature Transform (SIFT), Speeded Up Robust Features (SURF), Oriented FAST and Rotated BRIEF (ORB) can be utilized to extract the head feature points of the user from each frame in the first video data and determine the corresponding relationship of the head feature points in different frames. By performing feature matching based on the corresponding relationship, a motion trajectory of the user's head in the video can be tracked, which can further obtain the shape and motion information of the user's head. Three-dimensional modeling can be performed based on the shape and motion information to obtain a real three-dimensional model of the user's head including the predetermined head marker points (i.e., the first three-dimensional model). Similarly, a three-dimensional model of the head including the predetermined head marker points and the detection device marker point when the user's head is wearing the wearable apparatus (i.e., a second three-dimensional model) can be obtained.

In some examples, when performing the feature matching on the head feature points, a random sampling consensus (RANSAC) algorithm is further used to remove an incorrect matching point, to improve accuracy of feature matching.

At S13, relative position information between a detection device in the wearable apparatus and the user's head is obtained based on the first three-dimensional model and the second three-dimensional model.

In some embodiments of the present disclosure, the first three-dimensional model includes the predetermined head marker points of the user, and the second three-dimensional model includes the predetermined head marker points and the detection device marker point. Said obtaining the relative position information between the detection device in the wearable apparatus and the user's head based on the first three-dimensional model and the second three-dimensional model includes: registering the first three-dimensional model and the second three-dimensional model based on the predetermined head marker points to obtain a position of the detection device on a scalp surface of the user; and obtaining the relative position information based on the position of the detection device on the scalp surface of the user and the predetermined head marker points.

In this embodiment, as an implementation manner, said obtaining the relative position information based on the position of the detection device on the scalp surface of the user and the predetermined head marker points includes: determining three-dimensional coordinates of the position of the detection device on the scalp surface of the user and the predetermined head marker points; and obtaining the relative position information based on the three-dimensional coordinates and positions of the predetermined head marker points on the user's head.

In an exemplary embodiment of the present disclosure, when registering the first three-dimensional model and the second three-dimensional model, since the wearable apparatus has a thickness, a specific position of the detection device on the scalp surface can be obtained by projecting the detection device marker point in the second three-dimensional model onto a surface of the first three-dimensional model through registration. The three-dimensional coordinates of each detection device marker point and the predetermined head marker points can be obtained using tools such as Maya, Python, or Matlab. Since positions of the predetermined head marker points on the user's head are determined and fixed, a specific position of the detection device relative to the user's head (i.e. relative position information) can be obtained based on the three-dimensional coordinates of the detection device marker point and the predetermined head marker points.

At S14, the first three-dimensional model is registered with a predetermined standard spatial template, and a brain area position of the user corresponding to the detection device is obtained based on a registration result and the relative position information.

In some embodiments of the present disclosure, said registering the first three-dimensional model with the predetermined standard spatial template, and obtaining the brain area (that is, the brain functional areas) position of the user corresponding to the detection device based on the registration result and the relative position information includes: voxelizing the first three-dimensional model, and converting the voxelized first three-dimensional model into the same voxel scale as the predetermined standard spatial template; mapping voxels of the first three-dimensional model to the predetermined standard spatial template using a non-rigid registration method; and obtaining the brain area position of the user corresponding to the detection device based on a mapping result and the relative position information.

In an exemplary embodiment of the present disclosure, the first three-dimensional model can be voxelized, and the voxelized first three-dimensional model can be converted into the same voxel scale as the predetermined standard spatial template (such as MNI 152, ICBM series templates, and Colin 27) using MagicaVoxel software. By mapping voxels of a user's head model to the predetermined standard spatial template using the non-rigid registration method, the brain area position of the user corresponding to the detection device can be obtained, that is, the brain area localization can be achieved.

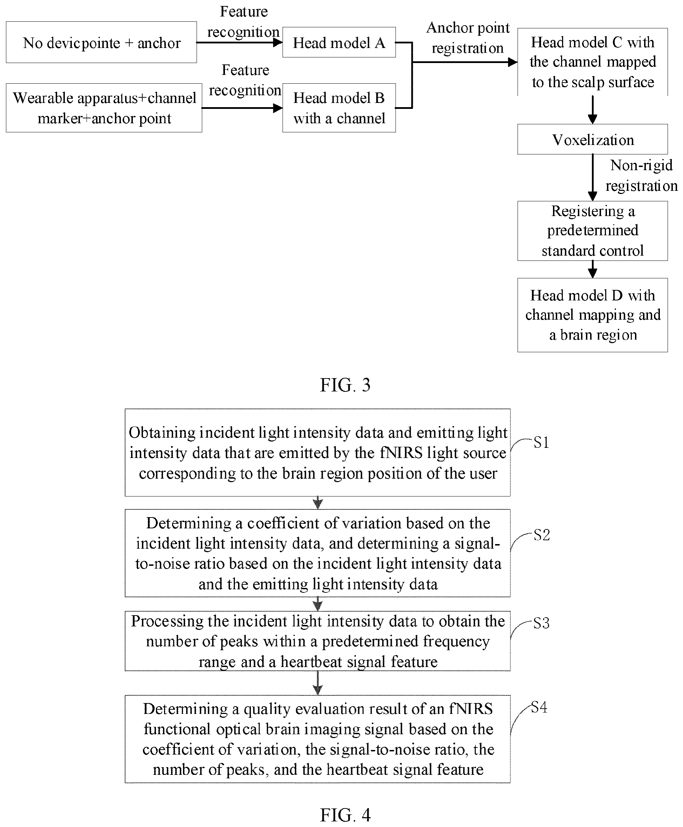

To facilitate understanding, the implementation process of the brain functional area location method of the present disclosure will be described below through a specific embodiment with reference to FIG. 3.

As illustrated in FIG. 3, the first video data is recorded based on "no device + anchor point", and feature recognition is performed on the first video data to obtain a "head model A" (i.e., the first three-dimensional model). "no device" means that the user's head is not wearing the wearable apparatus, and "anchor point" means that the predetermined head marker points are set. The second video data is recorded based on "wearable apparatus + channel marker + anchor point", and feature recognition is performed on the second video data to obtain a "head model B" with a channel (i.e., the second three-dimensional model). "Wearable apparatus" means that the user's head is wearing the wearable apparatus, "channel marker" means that the detection device marker point is set, and "anchor point" means that the predetermined head marker points are set.

Anchor point registration is performed on the head model A and the head model B to obtain a "head model C with channels mapped to the scalp surface" (including the above-described relative position information). Subsequently, the head model C is "voxelized", and then "registered to a predetermined standard control template" via the non-rigid registration method to obtain a "head model D with channel mapping and a brain area" (achieving the brain area localization of the detection device).

In the embodiments of the present disclosure, the real shape of the user's head is obtained by recording the multi-angle video of the user's head. Also, voxel registration is used in a process of brain area registration, which achieves higher precision compared with anchor point registration. Moreover, the method can be adapted to a variety of collection devices, has lower requirements for devices and low costs, and can be widely used.

The wearable apparatus as an fNIRS functional optical brain imaging device is taken as an example in the following to describe the application of determining the brain area position. For example, subsequent to determining the brain area position of the user corresponding to the detection device (such as the fNIRS light source) of the fNIRS functional optical brain imaging device using the method according to the above-described embodiments of the present disclosure, an fNIRS optical brain imaging signal of the brain area position can be collected and quality of the signal can be evaluated. As illustrated in FIG. 4, a method for evaluating quality of an fNIRS functional optical brain imaging signal in the brain functional area location method according to an embodiment of the present disclosure includes the operations.

At S1, incident light intensity data and emitting light intensity data that are emitted by the fNIRS light source corresponding to the brain region position of the user are obtained.

At S2, a coefficient of variation is determined based on the incident light intensity data, and a signal-to-noise ratio is determined based on the incident light intensity data and the emitting light intensity data.

At S3, the incident light intensity data is processed to obtain the number of peaks within a predetermined frequency range and a heartbeat signal feature.

At S4, a quality evaluation result of the fNIRS functional optical brain imaging signal is determined based on the coefficient of variation, the signal-to-noise ratio, the number of peaks, and the heartbeat signal feature.

In an exemplary embodiment of the present disclosure, the incident light intensity data and the emitting light intensity data that are emitted by the fNIRS light source are obtained. The incident light intensity data refers to intensity of light emitted from the fNIRS light source, that is, the light intensity measured before a to-be-measured object (such as the human head). This data is crucial for evaluating initial output of the fNIRS light source and propagation characteristics of the light. The emitting light intensity data refers to the light intensity after being absorbed and scattered by the to-be-measured object, that is, the light intensity measured after the to-be-measured object. The emitting light intensity data provides information about interaction between light and tissue, which is of great importance for evaluating penetration and absorption characteristics of the signal.

Subsequent to obtaining the incident light intensity data and emitting light intensity data, the coefficient of variation is determined based on the incident light intensity data, and the signal-to-noise ratio is determined based on the incident light intensity data and the emitting light intensity data. That is, the coefficient of variation is an indicator for measuring stability of the signal, and calculated as a ratio of a standard deviation to a mean. Based on the incident light intensity data, the coefficient of variation of the signal can be calculated to evaluate the stability and consistency of the signal. The signal-to-noise ratio is a ratio of signal intensity to background noise intensity and used to measure the quality of the signal. Based on the incident light intensity and emitting light intensity data, the signal-to-noise ratio can be determined, to evaluate proportion of useful information to noise in the signal.

The incident light intensity data can be processed to obtain the number of peaks within the predetermined frequency range and the heartbeat signal feature. For example, through frequency domain analysis of the incident light intensity data, the number of peaks within a specific frequency range (such as respiratory and heartbeat frequency ranges) can be identified. These peaks may be related to physiological activities, such as heartbeat and respiration, and their presence and quantity can provide additional information about signal quality. By analyzing these peaks, physiologically related signals and noise components present in the signal can be can identified. A high-quality signal should have an appropriate number of peaks, which are neither too much nor too few, indicating that the signal contains valid physiological information without being overwhelmed by noise. By analyzing the heartbeat-related signal in the incident light intensity data, the feature of the heartbeat signal, such as heartbeat frequency and amplitude, can be extracted. Such feature is helpful to evaluate information related to physiological activities in the signal and can be used to further analyze and optimize the signal quality.

In this way, subsequent to obtaining the coefficient of variation, the signal-to-noise ratio, the number of peaks, and the heartbeat signal feature, the quality evaluation result of the fNIRS functional optical brain imaging signal is determined based on the coefficient of variation, the signal-to-noise ratio, the number of peaks, and the heartbeat signal feature. That is, the coefficient of variation, the signal-to-noise ratio, the number of peaks, and the heartbeat signal feature are combined to comprehensively evaluate the quality of the fNIRS functional optical brain imaging signal. For example, the quality of the fNIRS functional optical brain imaging signal is evaluated according to each indicator. For example, each indicator can correspond to an evaluation result, such as a quality score. The quality score increases as the evaluation result becomes better. A comprehensive score can be calculated by weighting and summing these scores to determine a final quality evaluation result. That is, each indicator provides a different aspect of signal quality, and combining these indicators allows for more comprehensive evaluation of accuracy, stability, and physiological relevance of the signal. The final quality evaluation result is obtained based on comprehensive analysis of the above-described indicators and can be used to determine whether the signal is suitable for further analysis and application. If the quality evaluation result of the signal is lower than a predetermined threshold, an adjustment measure may need to be taken. For example, the signal quality is improved by adjusting the light intensity of the fNIRS light source or optimizing a signal processing method. Alternatively, the signal quality can be improved by enhancing detector sensitivity or improving optical path design.

In this way, it is possible to comprehensively measure the quality of the fNIRS functional optical brain imaging signal and also adapt to physiological characteristics of different individuals to guide real-time signal quality detection and hardware iterative design.

According to an embodiment of the present disclosure, the heartbeat signal feature includes an average value of inter-beat intervals, an average heart rate, a standard deviation of normal-to-normal intervals, as well as a cross-correlation coefficient and peak power between red light and infrared light corresponding to a heartbeat signal. Obtaining the heartbeat signal feature includes: converting the incident light intensity data into optical density data, filtering chromatic light at an infrared wavelength and performing R-point detection, and calculating the average value of inter-beat intervals, the average heart rate, and the standard deviation of normal-to-normal intervals by means of a sliding window approach.

In an exemplary embodiment of the present disclosure, the incident light intensity data refers to the intensity of light emitted from the fNIRS light source, while the optical density data refers to the intensity of light after passing through a tissue (such as the scalp and brain) and being absorbed and scattered by the tissue. By converting the incident light intensity data into the optical density data, the interaction between light and tissue can be reflected more accurately, which is a critical step in evaluating the quality of the fNIRS functional optical brain imaging signal. That is, in fNIRS, light at infrared wavelengths is used to monitor changes in cerebral hemodynamics. Filtering the light at the infrared wavelength is to remove irrelevant noise and interference and retain signal components related to heartbeat. This usually involves a band-pass filter to limit the signal to a specific frequency range, such as 0.5 Hz to 4.5 Hz, which is a main frequency range of heartbeat signals. R-point detection refers to detection of a peak point corresponding to the heartbeat in an electrocardiogram or similar biological signal. In the fNIRS, R point corresponds to a maximum point of light intensity change caused by heartbeat. By detecting such point, a heartbeat event can be accurately identified, laying a foundation for subsequent extraction of the heartbeat signal feature.

In this way, the average value of inter-beat intervals, the average heart rate, and the standard deviation of normal-to-normal intervals can be calculated by means of the sliding window approach. An Inter-Beat Interval (IBI) refers to a time interval between two consecutive heartbeats. By calculating the average value of a series of inter-beat intervals, an average inter-beat interval can be obtained, which is an important indicator for evaluating heart rate variability. The average heart rate refers to the average number of heartbeats per unit time, is usually expressed as the number of heartbeats per minute, and can be calculated by dividing 60,000 (the number of milliseconds in a minute) by the average inter-beat interval (in milliseconds). A standard deviation of normal to normal (SDNN) is an indicator for evaluating variability of inter-beat intervals and is obtained by calculating a standard deviation of differences between consecutive inter-beat intervals. The SDNN reflects overall heart rate variability. In this way, the heartbeat signal feature can be comprehensively obtained. Such feature is essential for evaluating the quality of the fNIRS functional optical brain imaging signal. Such feature not only reflect regularity and variability of heartbeat but also reveal a health state of a cardiovascular system and an activity state of an autonomic nervous system.

According to an embodiment of the present disclosure, said obtaining the number of peaks within the predetermined frequency range includes: performing Fourier transform on the incident light intensity data to obtain frequency domain signal of the incident light intensity data; ranking amplitudes of the frequency domain signal within the predetermined frequency range in descending order; and taking a predetermined number of amplitudes that are ranked top as peaks, and obtaining the number of the peaks. The predetermined number and predetermined range can be determined as desired.

In an exemplary embodiment of the present disclosure, when obtaining the number of peaks within the predetermined frequency range, the Fourier transform can be performed on the incident light intensity data to obtain the frequency domain signal of the incident light intensity data. The Fourier transform can efficiently calculate discrete Fourier transform and decompose the signal into a superposition of various frequency components. After the Fourier transform, a series of complex numbers are obtained. Each complex number represents a signal component at a specific frequency. The modulus (absolute value) of these complex numbers represents an amplitude of the signal at a corresponding frequency, and a phase angle represents a phase of the signal. A predetermined frequency range, such as the frequency range of heartbeat and respiration, is determined, for example, in advance based on the physiological characteristics of the fNIRS functional optical brain imaging signal. In this range, amplitudes of the corresponding frequency domain signal components are extracted and ranked in descending order. From the ranked amplitudes, the predetermined number of amplitudes that are ranked is selected as the peaks. These peaks represent the strongest frequency components in the signal, that is, signals of physiological activities such as heartbeat and respiration. In addition, the predetermined number can be determined based on the experimental design or analysis objectives. For example, if the focus is on the strongest heartbeat signals, serval peaks with the highest amplitudes can be selected, and the number of these peaks is the desired number of peaks. For example, the observable frequency range is 0 to fs/2. Within this frequency band range, five significant peaks are extracted, and frequency bands where these peaks are located are checked. If the frequency band is within the range of respiration, heartbeat, etc., it is considered that there is no significant interference from other frequency bands.

In this way, the main peaks within the predetermined frequency range can be extracted from the fNIRS functional optical brain imaging signal. These peaks reflect the most important physiological activity information in the signal and are crucial for evaluating signal quality and further analysis.

According to an embodiment of the present disclosure, said determining the quality evaluation result of the fNIRS functional optical brain imaging signal based on the coefficient of variation, the signal-to-noise ratio, the number of peaks, and the heartbeat signal feature includes: determining a first signal quality index based on the coefficient of variation, the coefficient of variation being negatively correlated with the first signal quality index; determining a second signal quality index based on the number of peaks and a predetermined number of peaks, a difference between the number of peaks and the predetermined number of peaks being negatively correlated with the second signal quality index; determining a third signal quality index based on the average value of inter-beat intervals, the average heart rate, and a first standard deviation of normal-to-normal intervals, an average value of reference inter-beat intervals, a reference average heart rate, and a second standard deviation of reference normal-to-normal intervals, and determining a fourth signal quality index based on a cross-correlation coefficient difference between the cross-correlation coefficient and a predetermined cross-correlation coefficient, a peak power difference between the peak power and a predetermined peak power threshold, wherein a target difference is determined using correlation and variance analysis based on the first standard deviation and the second standard deviation, the target difference being negatively correlated with the third signal quality index, and the cross-correlation coefficient difference and the peak power difference being each positively correlated with the fourth signal quality index; determining a fifth signal quality index based on the signal-to-noise ratio, the signal-to-noise ratio being positively correlated with the fifth signal quality index; determining a first product of the first signal quality index and a corresponding weight coefficient, a second product of the second signal quality index and a corresponding weight coefficient, a third product of the third signal quality index and a corresponding weight coefficient, a fourth product of the fourth signal quality index and a corresponding weight coefficient, and a fifth product of the fifth signal quality index and a corresponding weight coefficient; and determining the quality evaluation result of the fNIRS functional optical brain imaging signal based on a sum of the first product, the second product, the third product, the fourth product and the fifth product, wherein the evaluation result being positively correlated with each of the first signal quality index, the second signal quality index, the third signal quality index, the fourth signal quality index and the fifth signal quality index .

In an exemplary embodiment of the present disclosure, when determining the quality evaluation result of the fNIRS functional optical brain imaging signal based on the coefficient of variation, the signal-to-noise ratio, the number of peaks, and the heartbeat signal feature, the first signal quality index can first be determined based on the coefficient of variation. The coefficient of variation is negatively correlated with the first signal quality index. That is, the first signal quality index decreases as the coefficient of variation increases, and the first signal quality index increases as the coefficient of variation decreases. That is, the coefficient of variation is an indicator for measuring stability of the signal and defined as a ratio of the standard deviation to the mean. A higher value of the coefficient of variation indicates greater variability of the signal and poorer stability. Therefore, the coefficient of variation is negatively correlated with the first signal quality index. That is, the first signal quality index becomes higher as the coefficient of variation decreases.

Next, the second signal quality index is determined based on the number of peaks and the predetermined number of peaks. The difference between the number of peaks and the predetermined number of peaks is negatively correlated with the second signal quality index. That is, the second signal quality index decreases as the difference between the number of peaks and the predetermined number of peaks increases, and the second signal quality index increases as the difference between the number of peaks and the predetermined number of peaks decreases. That is, the number of peaks within the predetermined frequency range is extracted by performing Fourier transform on the incident light intensity data. A smaller difference between the number of peaks and the predetermined number of peaks indicates more valid physiological information contained in the signal. Therefore, the second signal quality index becomes higher.

Subsequently, the third signal quality index can be determined based on the average value of inter-beat intervals, the average heart rate, and the first standard deviation of normal-to-normal intervals, the average value of the reference inter-beat intervals, the reference average heart rate, and the second standard deviation of the reference normal-to-normal intervals. The average value of the reference inter-beat intervals, the reference average heart rate, and the reference normal-to-normal intervals can be obtained by collecting PPG signals using other heart rate devices (such as Photoplethysmogram (PPG)) and calculating R points overtime, thereby obtaining the average value of the reference inter-beat intervals, the reference average heart rate, and the second standard deviation of the reference normal-to-normal intervals. Smaller deviation between these indicators and the reference values indicates stronger physiological relevance of the signal. That is, the target difference can be determined by correlation and variance analysis. This target difference is negatively correlated with the third signal quality index. That is, a larger target difference corresponds to a lower third signal quality index, indicating that the quality of the fNIRS functional optical brain imaging signal is poor. Conversely, a smaller target difference corresponds to a higher third signal quality index, indicating that the quality of the fNIRS functional optical brain imaging signal is good.

Moreover, the fourth signal quality index can be determined based on the cross-correlation coefficient difference between the cross-correlation coefficient and the predetermined cross-correlation coefficient, and the peak power difference between the peak power and the predetermined peak power threshold. The cross-correlation coefficient and peak power are indicators for measuring correlation between red light and infrared light signal. The cross-correlation coefficient difference and the peak power difference are positively correlated with the fourth signal quality index. That is, smaller differences result in a higher fourth signal quality index, indicating that the quality of the fNIRS functional optical brain imaging signal is better. Conversely, larger differences result in a lower fourth signal quality index, indicating that the quality of the fNIRS functional optical brain imaging signal is poorer.

In addition, the fifth signal quality index can be determined based on the signal-to-noise ratio. The signal-to-noise ratio is positively correlated with the fifth signal quality index. That is, a larger signal-to-noise ratio indicates that the received signal contains more useful information components and fewer noise components and has better quality, resulting in a larger fifth signal quality index. Conversely, a smaller signal-to-noise ratio indicates that the received signal contains more noise components and has poorer quality, resulting in a smaller fifth signal quality index. In other words, the signal-to-noise ratio serves as a measure of the rationality of light intensity setting.

The above-described five signal quality indices are multiplied by the corresponding weight coefficients and then summed to obtain the quality evaluation result of the fNIRS functional optical brain imaging signal. That is, the first product of the first signal quality index and the corresponding weight coefficient, the second product of the second signal quality index and the corresponding weight coefficient, the third product of the third signal quality index and the corresponding weight coefficient, the fourth product of the fourth signal quality index and the corresponding weight coefficient, and the fifth product of the fifth signal quality index and the corresponding weight coefficient are determined, and the quality evaluation result of the fNIRS functional optical brain imaging signal is determined based on the sum of the first product, the second product, the third product, the fourth product, and the fifth product. Moreover, this evaluation result is positively correlated with each of the first signal quality index, the second signal quality index, the third signal quality index, the fourth signal quality index and the fifth signal quality index. That is, higher indices result in a better signal quality evaluation result, indicating that the accuracy and reliability of the signal are higher. In this way, the quality of the fNIRS functional optical brain imaging signal can be comprehensively evaluated, providing an important reference for subsequent data analysis and application.

According to an embodiment of the present disclosure, the method for evaluating the quality of the fNIRS functional optical brain imaging signal further includes: adjusting the signal-to-noise ratio when the quality evaluation result of the fNIRS functional optical brain imaging signal is smaller than a predetermined result threshold. The predetermined result threshold can be determined as desired.

In an exemplary embodiment of the present disclosure, the evaluation result can be determined based on a series of indicators, such as the coefficient of variation, the signal-to-noise ratio, the cross-correlation coefficient, and the peak power. For example, a better evaluation result corresponds to higher signal quality indices, and the quality of the evaluation result can be determined based on magnitudes of the signal quality indices. The predetermined result threshold refers to a standard value set prior to performing signal quality evaluation and is used to determine whether the signal quality reaches an acceptable level. This threshold is set based on experimental data, previous research, or performance standards of devices.

When the quality evaluation result of the fNIRS functional optical brain imaging signal is lower than a predetermined threshold, for example, when the current signal quality indices of the fNIRS functional optical brain imaging signal are low, it means that the signal may be excessively affected by noise interference, or the signal itself is not sufficiently stable and accurate. In this case, the signal-to-noise ratio needs to be adjusted to improve the signal quality. For example, by adjusting the light intensity of the fNIRS light source, the signal intensity can be changed, thereby affecting the signal-to-noise ratio. That is, increasing the light intensity of the fNIRS light source can improve the signal intensity. Conversely, reducing the light intensity of the fNIRS light source can reduce noise but may reduce detectability of the signal. In addition, various filtering techniques, such as a band-pass filter, can be applied to remove noise outside a specific frequency band and retain important physiological signals. Through the above-described adjustments, the quality of signal collection can be optimized, making the fNIRS functional optical brain imaging signal more accurate and stable, and improving the reliability of monitoring results.

Thus, to ensure the accuracy and reliability of the fNIRS functional optical brain imaging signal in practical applications, especially in neuroscience research and clinical diagnosis, high-quality signals are crucial for obtaining accurate information on cerebral hemodynamic changes. By dynamically adjusting the signal-to-noise ratio, external noise can be effectively suppressed, and the stability and accuracy of the signal can be improved.

Further, according to an embodiment of the present disclosure, said adjusting the signal-to-noise ratio includes: increasing intensity of infrared light emitted by the fNIRS light source.

In an exemplary embodiment of the present disclosure, the signal-to-noise ratio is one of the key indicators for measuring the signal quality. A level of the signal-to-noise ratio directly affects the detectability and accuracy of the signal. The light intensity of the fNIRS light source is an important factor affecting the signal-to-noise ratio, as the light intensity is directly related to the signal intensity and the level of background noise. When the quality evaluation result of the fNIRS functional optical brain imaging signal is lower than the predetermined threshold, it means that the signal may be excessively affected by noise interference, or the signal itself is not sufficiently stable and accurate. In this case, the signal-to-noise ratio can be improved by increasing the intensity of the infrared light emitted by the fNIRS light source.

That is, when adjusting the signal-to-noise ratio, the signal intensity can be improved by increasing the light intensity of the fNIRS light source, thereby suppressing the influence of noise to a certain extent and improving the detectability of the signal. This adjustment helps improve the quality of the signal, enabling it to meet or exceed the predetermined threshold. In addition, after adjusting the light intensity of the fNIRS light source, it is necessary to re-conduct the signal quality evaluation to ensure that the adjusted signal quality reaches the predetermined threshold, thereby verifying the effectiveness of the light intensity adjustment and ensuring actual improvement in the signal quality. In this way, the signal-to-noise ratio of the fNIRS functional optical brain imaging signal can be effectively adjusted and optimized, improving the accuracy and stability of the signal.

According to an embodiment of the present disclosure, the method for evaluating the quality of the fNIRS functional optical brain imaging signal further includes: obtaining head data of a test subject; and obtaining hair density based on the head data, and increasing the intensity of the infrared light emitted by the fNIRS light source when the hair density is high.

In an exemplary embodiment of the present disclosure, prior to implementing the method for evaluating the quality of the fNIRS functional optical brain imaging signal, the head data of the test subject needs to be obtained. Such data may include information such as the shape and size of the head, as well as the hair density. This information is critical for the subsequent adjustment of the light intensity of the fNIRS light source, as it directly affects the ability of near-infrared light to penetrate the scalp and skull. The hair density is an important factor affecting the quality of the fNIRS functional optical brain imaging signal. An area with denser hair can absorb or scatter more near-infrared light, reducing the intensity and quality of the signal. Therefore, evaluating the hair density is essential for determining the appropriate light intensity of the fNIRS light source. When the evaluation results show that the hair density of the test subject is high, to ensure that the near-infrared light can effectively penetrate the scalp and skull and reach the cerebral cortex, it is necessary to increase the intensity of the infrared light emitted by the fNIRS light source. In this way, the strength of the signal can be increased, improving the signal-to-noise ratio and optimizing the signal quality. That is, increasing the light intensity of the fNIRS light source can help overcome the additional scattering and absorption caused by the higher density of hair, which ensures that enough light energy reaches the detector, obtaining more accurate information on changes in cerebral blood oxygen concentration.

In addition, subsequent to adjusting the light intensity of the fNIRS light source, the quality of the fNIRS functional optical brain imaging signal needs to be re-evaluated to ensure that the adjusted signal quality reaches the predetermined threshold. This step is essential to verify the effectiveness of the light intensity adjustment and ensure actual improvement in the signal quality. In this way, the method for evaluating the quality of the fNIRS functional optical brain imaging signal can adapt to the physiological characteristics of different individuals, especially in the case of high hair density. The quality of signal collection is optimized by adjusting the light intensity of the fNIRS light source, improving the reliability of monitoring results and the performance of the devices.

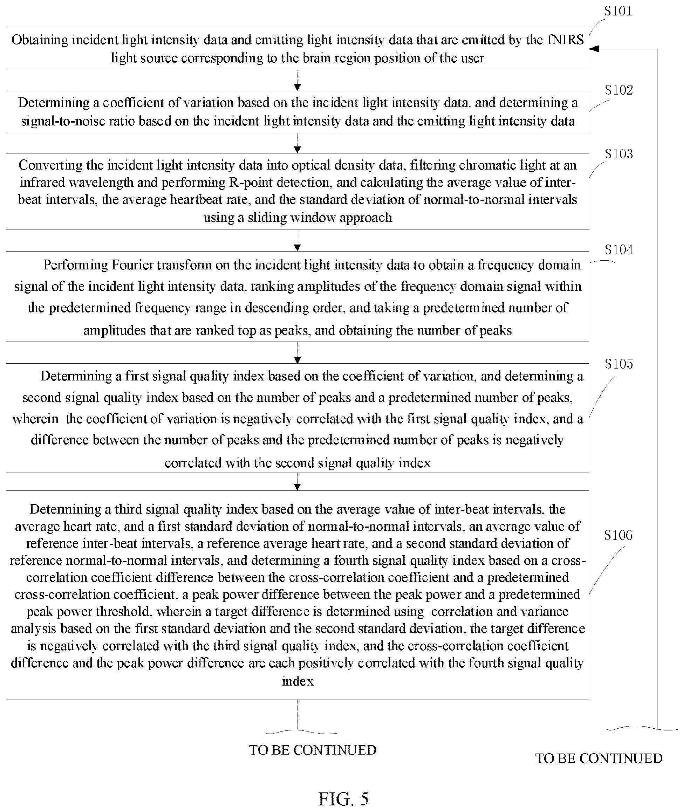

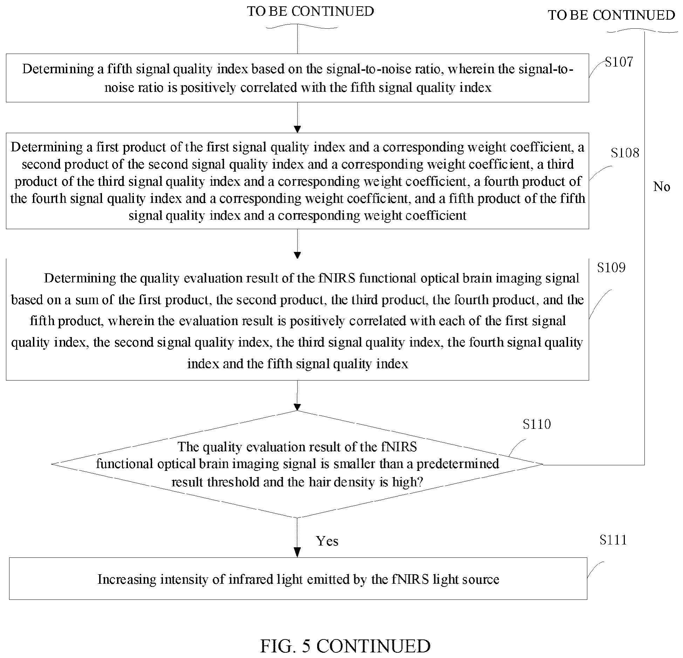

As a specific example, as illustrated in FIG. 5, another method for evaluating quality of an fNIRS functional optical brain imaging signal in a brain functional area location method according to an embodiment of the present disclosure includes the operations at blocks.

At S101, incident light intensity data and emitting light intensity data that are emitted by the fNIRS light source corresponding to the brain area position of the user are obtained.

At S102, a coefficient of variation is determined based on the incident light intensity data, and a signal-to-noise ratio is determined based on the incident light intensity data and the emitting light intensity data.

At S103, the incident light intensity data is converted into optical density data, chromatic light at an infrared wavelength is filtered and R-point detection is performed, and the average value of inter-beat intervals, the average heart rate, and the standard deviation of normal-to-normal intervals are calculated by means of a sliding window approach.

At S104, Fourier transform is performed on the incident light intensity data to obtain frequency domain signal of the incident light intensity data, amplitudes of the frequency domain signal within the predetermined frequency range are ranked in descending order, and the top predetermined number of amplitudes is taken as peaks, and the number of peaks is obtained.

At S105, a first signal quality index is determined based on the coefficient of variation, and a second signal quality index is determined based on the number of peaks and a predetermined number of peaks. The coefficient of variation is negatively correlated with the first signal quality index. A difference between the number of peaks and the predetermined number of peaks is negatively correlated with the second signal quality index.

At S106, a third signal quality index is determined based on the average value of inter-beat intervals, the average heart rate, and a first standard deviation of normal-to-normal intervals, an average value of reference inter-beat intervals, a reference average heart rate, and a second standard deviation of reference normal-to-normal intervals, and a fourth signal quality index is determined based on a cross-correlation coefficient difference between the cross-correlation coefficient and a predetermined cross-correlation coefficient, a peak power difference between the peak power and a predetermined peak power threshold. A target difference is determined using correlation and variance analysis based on the first standard deviation and the second standard deviation, and the target difference is negatively correlated with the third signal quality index. The cross-correlation coefficient difference and the peak power difference are each positively correlated with the fourth signal quality index.

At S107, a fifth signal quality index is determined based on the signal-to-noise ratio. The signal-to-noise ratio is positively correlated with the fifth signal quality index.

At S108, a first product of the first signal quality index and a corresponding weight coefficient, a second product of the second signal quality index and a corresponding weight coefficient, a third product of the third signal quality index and a corresponding weight coefficient, a fourth product of the fourth signal quality index and a corresponding weight coefficient, and a fifth product of the fifth signal quality index and a corresponding weight coefficient are determined.

At S109, the quality evaluation result of the fNIRS functional optical brain imaging signal is determined based on a sum of the first product, the second product, the third product, the fourth product, and the fifth product. The evaluation result is positively correlated with each of the first signal quality index, the second signal quality index, the third signal quality index, the fourth signal quality index and the fifth signal quality index.

At S110, it is determined whether the quality evaluation result of the fNIRS functional optical brain imaging signal is smaller than a predetermined result threshold and whether the hair density of a test subject is thick. If the quality evaluation result of the fNIRS functional optical brain imaging signal is smaller than a predetermined result threshold and the hair density is high, block S111 is executed. If the quality evaluation result of the fNIRS functional optical brain imaging signal is not smaller than a predetermined result threshold and the hair density is not thick, block S101 is executed.

At S111, the intensity of the infrared light emitted by the fNIRS light source is increased.

In summary, in the embodiments of the present disclosure, incident light intensity data and emitting light intensity data that are emitted by the fNIRS light source corresponding to the brain area position can be obtained. The coefficient of variation is determined based on the incident light intensity data, and the signal-to-noise ratio is determined based on the incident light intensity data and the emitting light intensity data. The incident light intensity data is processed to obtain the number of peaks within the predetermined frequency range and the heartbeat signal feature. The quality evaluation result of the fNIRS functional optical brain imaging signal is determined based on the coefficient of variation, the signal-to-noise ratio, the number of peaks, and the heartbeat signal feature. In this way, this method can comprehensively measure the quality of the near-infrared signal and adapt to the physiological characteristics of different individuals to guide real-time signal quality detection and hardware iterative design.

Corresponding to the above-described embodiments, the present disclosure further provides a computer-readable storage medium.

In this embodiment, the computer-readable storage medium has a computer program stored thereon. The computer program, when executed by a processor, implements the brain functional area location method according to the above-described embodiments.

Based on the above-described embodiments, the present disclosure further provides a wearable edge computing device.

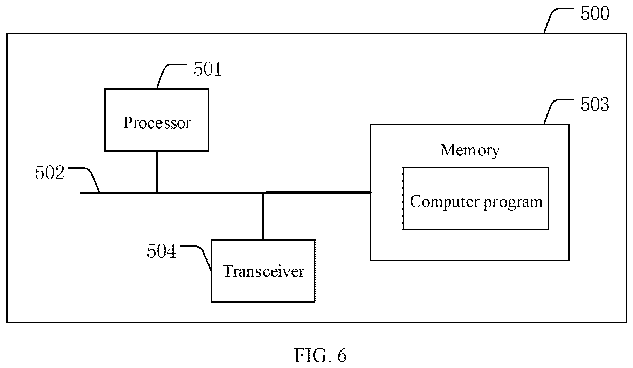

As illustrated in FIG. 6, a wearable edge computing device 500 includes a processor 501 and a memory 503. The processor 501 is connected to the memory 503, for example, via a bus 502. In another exemplary embodiment of the present disclosure, the wearable edge computing device 500 may further include a transceiver 504. It should be noted that in practical application, the wearable edge computing device 500 is not limited to include one transceiver 504, and a structure of the wearable edge computing device 500 does not constitute a limitation on the embodiments of the present disclosure.

The processor 501 may be a Central Processing Unit (CPU), a general-purpose processor, a Digital Signal Processor (DSP), an Application Specific Integrated Circuit (ASIC), a Field-Programmable Gate Array (FPGA) or other programmable logic devices, transistor logic devices, hardware components, or any combination thereof, which can realize or execute various exemplary logic blocks, modules, and circuits described in the present disclosure. The processor 501 can also be a combination that achieves a computing function, such as a combination of one or more microprocessors, a combination of a DSP and a microprocessor, etc.

The bus 502 can include a pathway for transmitting information between the above components. The bus 502 can be a PCI (Peripheral Component Interconnect) bus, or an EISA (Extended Industry Standard Architecture) bus, etc. The bus 502 can be divided into an address bus, a data bus, a control bus, etc. For ease of representation, only one thick line is used in FIG. 6, but it does not mean that there is only one bus or one type of bus.

Memory 503 stores a computer program corresponding to the brain functional area location method according to the above embodiments of the present disclosure. The computer program is controlled and executed by the processor 501. The processor 501 is configured to execute a computer program stored in the memory 503 to implement the method embodiments shown above.

The wearable edge computing device 500 may include, but is not limited to, a wearable electroencephalographic (EEG) edge computing device, a wearable fNIRS functional optical brain imaging edge computing device, a wearable diffusion optical tomography (DOT) edge computing device, and other types of edge computing devices that are wearable on the head. The wearable edge computing device 500 shown in FIG. 6 is merely an example and should not impose any limitation to the functions and scope of use of the embodiments of the present disclosure.

It should be noted that the logic and/or step described in other manners herein or shown in the flow chart, for example, a particular sequence list of executable instructions for realizing the logical function, may be specifically achieved in any computer readable medium to be used by the instruction execution system, device or equipment (such as the system based on computers, the system comprising processors or other systems capable of obtaining the instruction from the instruction execution system, device and equipment and executing the instruction), or to be used in combination with the instruction execution system, device and equipment. As to the specification, "the computer readable medium" may be any device adaptive for including, storing, communicating, propagating or transferring programs to be used by or in combination with the instruction execution system, device or equipment. More specific examples of the computer readable medium comprise but are not limited to: an electronic connection (an electronic device) with one or more wires, a portable computer enclosure (a magnetic device), a random access memory (RAM), a read only memory (ROM), an erasable programmable read-only memory (EPROM or a flash memory), an optical fiber device and a portable compact disk read-only memory (CDROM). In addition, the computer readable medium may even be a paper or other appropriate medium capable of printing programs thereon, this is because, for example, the paper or other appropriate medium may be optically scanned and then edited, decrypted or processed with other appropriate methods when necessary to obtain the programs in an electric manner, and then the programs may be stored in the computer memories.

It should be understood that each part of the present disclosure may be realized by hardware, software, firmware, or a combination thereof. In the above embodiments, a plurality of steps or methods may be realized by the software or firmware stored in the memory and executed by the appropriate instruction execution system. For example, if it is realized by the hardware, likewise in another embodiment, the steps or methods may be realized by one or a combination of the following techniques known in the art: a discrete logic circuit having a logic gate circuit for realizing a logic function of a data signal, an application-specific integrated circuit having an appropriate combination logic gate circuit, a programmable gate array (PGA), a field programmable gate array (FPGA), etc.

Reference throughout this specification to "an embodiment", "some embodiments", "an example", "a specific example", or "some examples" means that a particular feature, structure, material, or characteristic described in connection with the embodiment or example is included in at least one embodiment or example of the present disclosure. In this specification, exemplary descriptions of aforesaid terms are not necessarily referring to the same embodiment or example. Moreover, the described specific features, structures, materials or characteristics may be combined in any one or more embodiments or examples in a suitable manner.

In the description of the present disclosure, it should be understood that the orientation or the position indicated by terms such as "center", "longitudinal", "transverse", "length", "width", "thickness", "over", "below", "front", "rear", "left", "right", "vertical", "horizontal", "top", "bottom", "inner", "outer", "clockwise", "anti-clockwise", "axial", "radial", and "circumferential" should be construed to refer to the orientation or the position as shown in the drawings, and is only for the convenience of describing the present disclosure and simplifying the description, rather than indicating or implying that the pointed device or element must have a specific orientation, or be constructed and operated in a specific orientation, and therefore cannot be understood as a limitation of the present disclosure.

In addition, terms "first" and "second" are only used for descriptive purposes, and cannot be understood as indicating or implying relative importance or implicitly indicating the number of indicated technical features. Therefore, the features defined with "first" and "second" may explicitly or implicitly include at least one of the features. In the description of the present disclosure, "a plurality of' means at least two, for example, two or three, unless specified otherwise.

In the present disclosure, unless otherwise clearly specified and limited, terms such as "install", "connect", "connect to", "fix", and the like should be understood in a broad sense. For example, it may be a fixed connection or a detachable connection or connection as one piece; mechanical connection or electrical connection or communication; direct connection or indirect connection through an intermediate; internal communication of two components or the interaction relationship between two components, unless otherwise specifically defined. For those of ordinary skill in the art, the specific meaning of the above-mentioned terms in the present disclosure can be understood according to specific circumstances.

In the present disclosure, unless specified or limited otherwise, the first characteristic is "on" or "under" the second characteristic refers to the first characteristic and the second characteristic can be direct or via media indirect mountings, connections, and couplings. And, the first characteristic is "on", "above", "over" the second characteristic may refer to the first characteristic is right over the second characteristic or is diagonal above the second characteristic, or just refer to the horizontal height of the first characteristic is higher than the horizontal height of the second characteristic. The first characteristic is "below" or "under" the second characteristic may refer to the first characteristic is right over the second characteristic or is diagonal under the second characteristic, or just refer to the horizontal height of the first characteristic is lower than the horizontal height of the second characteristic.

Although embodiments of the present disclosure have been illustrated and described above, it should be understood that the above embodiments are merely exemplary, and cannot be construed to limit the present disclosure. For those skilled in the art, changes, modifications, substitutions, and variations can be made to the embodiments without departing from the scope of the present disclosure.

## Claims

1. A brain functional area location method, comprising:
obtaining first video data and second video data, wherein the first video data is a multi-angle head video recorded when a user's head is not wearing a wearable apparatus, and wherein the second video data is a multi-angle head video recorded when the user's head is wearing the wearable apparatus;
obtaining a first three-dimensional model of the user's head based on the first video data, and obtaining a second three-dimensional model of the user's head based on the second video data;
obtaining relative position information between a detection device in the wearable apparatus and the user's head based on the first three-dimensional model and the second three-dimensional model; and
registering the first three-dimensional model with a predetermined standard spatial template, and obtaining a brain area position of the user corresponding to the detection device based on a registration result and the relative position information.

2. The method according to claim 1, wherein said obtaining the first three-dimensional model of the user's head based on the first video data comprises:
extracting head feature points of the user from each frame in the first video data;
performing feature matching on the head feature points to obtain a shape and motion information of the user's head; and
obtaining the first three-dimensional model based on the shape and the motion information.

3. The method according to claim 2, wherein when performing the feature matching on the head feature points, a random sampling consensus algorithm is further used to remove incorrect matching points.

4. The method according to claim 1, wherein the first three-dimensional model comprises predetermined head marker points of the user, and the second three-dimensional model comprises the predetermined head marker points and a detection device marker point; said obtaining the relative position information between the detection device in the wearable apparatus and the user's head based on the first three-dimensional model and the second three-dimensional model comprising:
registering the first three-dimensional model and the second three-dimensional model based on the predetermined head marker points to obtain a position of the detection device on a scalp surface of the user; and
obtaining the relative position information based on the position of the detection device on the scalp surface of the user and the predetermined head marker points.

5. The method according to claim 4, wherein said obtaining the relative position information based on the position of the detection device on the scalp surface of the user and the predetermined head marker points comprises:
determining three-dimensional coordinates of the position of the detection device on the scalp surface of the user and the predetermined head marker points; and
obtaining the relative position information based on the three-dimensional coordinates and positions of the predetermined head marker points on the user's head.

6. The method according to claim 4 or claim 5, wherein the predetermined head marker points comprise at least two of a nasion position, bilateral ear canal mastoid positions, and a posterior cranial protuberance position of the user.

7. The method according to claim 1, wherein said registering the first three-dimensional model with the predetermined standard spatial template, and obtaining the brain area position of the user corresponding to the detection device based on the registration result and the relative position information comprises:
voxelizing the first three-dimensional model, and converting the voxelized first three-dimensional model into the same voxel scale as the predetermined standard spatial template;
mapping voxels of the first three-dimensional model to the predetermined standard spatial template using a non-rigid registration method; and
obtaining the brain area position of the user corresponding to the detection device based on a mapping result and the relative position information.

8. The method according to any one of claims 1 to 7, wherein the wearable apparatus comprises a wearable fNIRS functional optical brain imaging device, the detection device comprises an fNIRS light source, the method further comprising:
obtaining incident light intensity data and emitting light intensity data that are emitted by the fNIRS light source corresponding to the brain area position of the user;
determining a coefficient of variation based on the incident light intensity data, and determining a signal-to-noise ratio based on the incident light intensity data and the emitting light intensity data;
processing the incident light intensity data to obtain the number of peaks within a predetermined frequency range and a heartbeat signal feature; and
determining a quality evaluation result of an fNIRS functional optical brain imaging signal based on the coefficient of variation, the signal-to-noise ratio, the number of peaks, and the heartbeat signal feature.

9. The method according to claim 8, wherein the heartbeat signal feature comprises an average value of inter-beat intervals, an average heart rate, a standard deviation of normal-to-normal intervals, as well as a cross-correlation coefficient and peak power between red light and infrared light corresponding to a heartbeat signal, said obtaining the heartbeat signal feature comprising:
converting the incident light intensity data into optical density data, filtering chromatic light at an infrared wavelength and performing R-point detection, and calculating the average value of inter-beat intervals, the average heart rate, and the standard deviation of normal-to-normal intervals using a sliding window approach.

10. The method according to claim 9, wherein said obtaining the number of peaks within the predetermined frequency range comprises:
performing Fourier transform on the incident light intensity data to obtain frequency domain signal of the incident light intensity data;
ranking amplitudes of the frequency domain signal within the predetermined frequency range in descending order; and
taking a predetermined number of amplitudes that are ranked top as peaks, and obtaining the number of peaks.

11. The method according to claim 10, wherein said determining the quality evaluation result of the fNIRS functional optical brain imaging signal based on the coefficient of variation, the signal-to-noise ratio, the number of peaks, and the heartbeat signal feature comprises:
determining a first signal quality index based on the coefficient of variation, wherein the coefficient of variation is negatively correlated with the first signal quality index;
determining a second signal quality index based on the number of peaks and a predetermined number of peaks, wherein a difference between the number of peaks and the predetermined number of peaks is negatively correlated with the second signal quality index;
determining a third signal quality index based on the average value of inter-beat intervals, the average heart rate, and a first standard deviation of normal-to-normal intervals, an average value of reference inter-beat intervals, a reference average heart rate, and a second standard deviation of reference normal-to-normal intervals, and determining a fourth signal quality index based on a cross-correlation coefficient difference between the cross-correlation coefficient and a predetermined cross-correlation coefficient, a peak power difference between the peak power and a predetermined peak power threshold, wherein a target difference is determined using correlation and variance analysis based on the first standard deviation and the second standard deviation, the target difference is negatively correlated with the third signal quality index, and the cross-correlation coefficient difference and the peak power difference are each positively correlated with the fourth signal quality index;
determining a fifth signal quality index based on the signal-to-noise ratio, wherein the signal-to-noise ratio is positively correlated with the fifth signal quality index;
determining a first product of the first signal quality index and a corresponding weight coefficient, a second product of the second signal quality index and a corresponding weight coefficient, a third product of the third signal quality index and a corresponding weight coefficient, a fourth product of the fourth signal quality index and a corresponding weight coefficient, and a fifth product of the fifth signal quality index and a corresponding weight coefficient; and
determining the quality evaluation result of the fNIRS functional optical brain imaging signal based on a sum of the first product, the second product, the third product, and the fourth product and the fifth product, wherein the evaluation result is positively correlated with each of the first signal quality index, the second signal quality index, the third signal quality index, the fourth signal quality index and the fifth signal quality index.

12. The method according to claim 9, further comprising: adjusting the signal-to-noise ratio when the quality evaluation result of the fNIRS functional optical brain imaging signal is smaller than a predetermined result threshold.

13. The method according to claim 12, wherein said adjusting the signal-to-noise ratio comprises:
increasing intensity of infrared light emitted by the fNIRS light source.

14. A wearable edge computing device, comprising:
a memory; and
a processor;
wherein the memory has a computer program stored thereon, and wherein the processor implements, when executing the computer program, the method according to any one of claims 1 to 13.

15. A computer-readable storage medium, having a computer program stored thereon, wherein the computer program, when executed by a processor, implements the method according to any one of claims 1 to 13.
